# EUROPEAN PATENT APPLICATION

(11) **EP 2 765 186 A1**
(43) Date of publication of application: **13.08.2014**
(21) Application number: 12792072.6
(22) Date of filing: 31.05.2012
(51) Int. Cl.: C12N 5/0775, A61L 27/38

(54) **STRUCTURE FOR TISSUE REGENERATION AND A PRODUCTION METHOD THEREFOR**

(30) Priority: 31.05.2011 KR 20110052036
(71) Applicant: SNU R&DB Foundation, Seoul 151-742 (KR)
(72) Inventor: CHO, Jae Jin, Seoul 150-748 (KR)
(74) Representative: Gardner, Rebecca Katherine
(86) International application number: PCT/KR2012/004316
(87) International publication number: WO 2012/165885

(57) **Abstract**

The present invention relates to a structure for tissue regeneration and to a production method therefor. The structure for tissue regeneration according to the present invention allows autologous tissue to be used, makes stable transplantation possible and can be produced without additional materials such as biopolymers. Also, it can be produced to a diameter of any desired size from a number of microns to a number of centimeters and can easily be produced in volume, and it contains large amounts of the extracellular matrix GAG, collagen and the like and can be used in tissue regeneration or filling in various fields including dermatology, plastic surgery, dentistry, surgery, orthopaedic surgery, urology, otolaryngology or in obstetrics and gynaecology.

## Description

### [Technical Field]

The present invention relates to a scaffold for tissue regeneration, comprising differentiated derivatives of mesenchymal stem cells, and a method for preparing the same.

### [Background Art]

Wounds can be divided, depending on the presence or absence of a break in the surface of the skin, into open wounds where the skin or mucous membrane is damaged and the internal tissue is exposed to the air, such as incised wound, lacerated wound, penetrating wound, abrasion, etc., and closed wounds where there is no break in the skin or mucous membrane but the underlying tissues are damaged, such as contusion, bruise, abscess, hematoma, etc.

The wound-healing process involves proliferation of epidermal cells such as fibroblasts, vascular endothelial cells, and keratinocytes, migration of cells to the wound site, granulation tissue formation, angiogenesis, reepithelialization, and tissue regeneration.

Wound-healing factors may include growth factors such as epidermal growth factor (EGF), acid fibroblast growth factor (aFGF), basic fibroblast growth factor (bFGF), transforming growth factor (TGF), and insulin-like growth factor (IGF), adhesion factors such as fibronectin, laminin, and vitronectin, and compounds such as retinoid, etc. (Thiry et al., Exp. Cell Res., 164:1, 1986; McGee et al., J. Surg. Res., 45:145, 1988; JP Publication No.2-167231; Sporn et al., Science, 233:532,1986; Travis and Salvesen, Ann. Rev. Biochem., 52:665, 1983; Ubels et al., Am. J. Ophthalmol., 95:353, 1983; Kastl et al., Ann. Ophthal., 19:175, 1987).

During the wound-healing process, a balance between anabolic and catabolic processes occurs for 6 to 8 weeks after the occurrence of a wound, and during this phase, a recovery of 30 to 40% normal skin tissues occurs. A scar is formed in a congestive, elevated state with increased tensile strength as collagen fibers are gradually crossed linked, but will be in a condition similar to that of the skin over time. During the wound-healing process, when an imbalance between anabolic and catabolic processes occurs, the collagen is not broken down but hardened, resulting in an elevated scar, and this tissue may form hypertrophic scar or keloids.

The interest in the wound healing and the development of various surgical procedures or drugs ultimately are aimed at preventing the occurrence of scars. Scars can be treated with surgical or nonsurgical means. The nonsurgical means may include occlusive dressings such as silicone gel sheets, Cordran tape, and Scarguard, treatment using pressure, steroid treatment for preventing collagen formation, etc., and the surgical means may include cryotherapy, laser, surgical incision, etc. It is known that the surgical treatment generally has a scar recurrence rate of 25 to 100% and thus is not effective. In addition, there is a method of applying interferon, verapamil, bleomycin, 5-fluorouracil (5-FU), retinoic acid, imiquimod, tacrolimus, or botulinum toxin topically to the scar site, but this method has low treatment efficiency and high reoccurrence rate and is limited in use due to side effects caused by drug administration.

Extensive research on tissue engineering of bone marrow-derived multipotent cells or stem cells for tissue regeneration has recently been conducted (Bi et al., J. Am. Soc. Nephrol., 18(9):2486, 2007; Wagatsuma A, Mol. Cell Biochem. 304(1-2):25, 2007; Song et al., Int. J. Impot. Res., 19(4):378, 2007).

In general, it is widely known that multipotent cells or stem cells are generally obtained from bone marrow, but the harvest of bone marrow is not easy, and the transplantation of stem cells from another person causes immune rejection. Therefore, adipose tissue is used as an alternative source of stem cells to solve these problems (Zuk et al., Tissue Eng., 7:211, 2001; Mizuno et al., Plast. Reconstr. Surg., 109:199, 2002; Zuk et al., Mol. Biol. Cel., 13:4279, 2002).

By non-invasive techniques such as liposuction, a large amount of adipose tissue can be obtained, and adipose tissue-derived stromal cells are found in the fat obtained by liposuction. These cells can be easily obtained from human adipose tissue degraded by collagenase and fractionated by differential centrifugation. Moreover, it is proved that these cells can differentiate into adipocytes, chondrocytes, osteocytes, etc. under appropriate culture conditions, because the expression of cell surface proteins is not identical, but similar to bone marrow stem cells (Erickson et al., Biochem. Biophys. Res. Commun., 290:763, 2002; Halvorsen et al., Tissue Eng., 7:729, 2001; Wickham et al., Clin. Orthop. Relat. Res., 412:196, 2003; Dragoo et al., J. Bone Joint Surg., Br., 85:740, 2003).

Stromal cells in adipose tissue are present in an amount of 8.6 x 10⁴ per gram of adipose tissue, and thus a sufficient amount of stromal cells can be obtained, unlike stem cells. Moreover, it is known that stromal cells can be supplied in quantities due to their ease of in vitro culture and can be used for tissue regeneration due to their ability to survive cryopreservation (US Patent No. 6,673,606).

As well as differentiation of cells, successful angiogenesis is necessary for tissue regeneration. Therapeutic angiogenesis developed for the treatment of ischemia serves to promote blood vessel development in ischemic tissue and thus can be applied to tissue regeneration. It is expected that these techniques can also be applied to reconstruction of tissues such as skin from a broader viewpoint, and the use of umbilical cord-derived stem cells is also contemplated.

Accordingly, the present inventors have made an effort to develop a scaffold for tissue regeneration and found that a scaffold, which is obtained by inducing aggregation of mesenchymal stem cells by applying a mechanical stress to the mesenchymal stem cells, without the use of any additional substances such as biopolymers, and culturing the aggregated mesenchymal stem cells, has excellent tissue regeneration properties, thus completing the present invention.

### [Disclosure]

### [Technical Problem]

Therefore, an object of the present invention is to provide a scaffold for tissue regeneration, comprising differentiated derivatives of mesenchymal stem cells, and a method for preparing the same.

### [Technical Solution]

To achieve the above object, the present invention provides a scaffold for tissue regeneration, comprising differentiated derivatives of mesenchymal stem cells, and a method for preparing the same.

### [Advantageous Effects]

The scaffold for tissue regeneration according to the present invention can be reliably transplanted with the use of autologous tissue and can be prepared without the use of any additional substances such as biopolymers. Moreover, it can be prepared with a diameter between several micrometers to several centimeters and can be easily prepared in quantities. Furthermore, it contains large amounts of extracellular matrices such as GAG, collagen, etc. and thus can be used for tissue regeneration, fillers, volumizers, restorative composites, etc. in various fields such as dermatology, plastic surgery, dentistry, surgery, orthopedics, urology, otolaryngology, obstetrics & gynaecology, etc.

### [Description of Drawings]

FIG. 1 shows images of adipose-derived mesenchymal stem cells stained with H&E, (SMA), PDGFR, and CD146.
FIG. 2 shows human adipose-derived mesenchymal stem cells at passages 1 and 2.
FIG. 3 shows a TEM image of adipose-derived mesenchymal stem cells.
FIG. 4 shows the distribution of cell number according to cells size.
FIG. 5 shows the analysis of markers for adipose-derived mesenchymal stem cells.
FIG. 6 shows the FACS analysis of cell surface antigens on adipose-derived mesenchymal stem cells.
FIG. 7 is a schematic diagram showing a method for preparing a scaffold for tissue regeneration according to the present invention.
FIG. 8 shows different sizes of scaffolds for tissue regeneration according to the present invention.
FIG. 9 shows images of scaffolds for tissue regeneration according to the present invention stained with H&E, Alcian Blue, Safranin-O, Collagen I, and Collagen II.
FIG. 10 shows the GAG content in a scaffold for tissue regeneration according to the present invention.
FIG. 11 shows the analysis of genes in a scaffold for tissue regeneration according to the present invention.

### [Best Mode for Carrying Out the Invention]

The present invention provides a method for preparing a scaffold for tissue regeneration, the method comprising the steps of: (a) inducing aggregation of mesenchymal stem cells by applying a mechanical stress to the mesenchymal stem cells; and (b) obtaining a scaffold containing both differentiated derivatives in a spherical form and mesenchymal stem cells by culturing the aggregated mesenchymal stem cells.

Moreover, the present invention provides a scaffold for tissue regeneration prepared by the above method.

Hereinafter, the present invention will be described in detail.

Step (a) is to induce aggregation of mesenchymal stem cells by applying a mechanical stress to the mesenchymal stem cells, and the mechanical stress may preferably be 1G to 500G.

The mesenchymal stem cells may be autologous mesenchymal stem cells or allogeneic mesenchymal stem cells and may be derived from adipose tissue, umbilical cord, or bone marrow, preferably derived from adipose tissue.

The adipose-derived stem cells are autologous or allogeneic and may include adipose stem cells and mesenchymal stem cells. The umbilical cord-derived stem cells are autologous or allogeneic and may include umbilical cord-derived mesenchymal stem cells. The bone marrow-derived stem cells are autologous or allogeneic whole blood cells and may include hematopoietic stem cells, mesenchymal stem cells, and endothelial progenitor cells.

Step (b) is to obtain a scaffold containing both differentiated derivatives in a spherical form and mesenchymal stem cells by culturing the aggregated mesenchymal stem cells, in which a bioactive substance may be further added to promote the induction of differentiation during the culture. The bioactive substance may comprise at least one additive selected from the group consisting of fibroblast growth factor (FGF), epidermal growth factor (EGF), platelet-derived growth factor (PDGF), connective tissue growth factor (CTGF), bone morphogenic protein (BMP), lipopolysaccharide (LPS), tumor necrosis factor-α (TNF-α), transforming growth factor-α (TGF-α), transforming growth factor-β (TGF-β), ascorbic acid, and dexamethasone, but not limited thereto.

In step (b), the differentiated derivatives may comprise fibroblasts.

Before step (a), the method may further comprise the step of activating the aggregation of mesenchymal stem cells.

After transplantation, the scaffold for tissue regeneration may be subjected to further treatment with matrix metalloproteinase (MMP) to remove the scaffold.

The scaffold for tissue regeneration prepared by the above method may preferably have a diameter of 1 µm to 10 cm.

The scaffold for tissue regeneration according to the present invention can be reliably transplanted with the use of autologous tissue and can be prepared without the use of any additional substances such as biopolymers. Moreover, it can be prepared with a diameter between several micrometers to several centimeters and can be easily prepared in quantities. Furthermore, it contains large amounts of extracellular matrices such as GAG, collagen, etc.

Therefore, the scaffold for tissue regeneration according to the present invention can be used for the treatment of wrinkles due to tissue aging, sagging skins, volumizing, etc. More specifically, the scaffold for tissue regeneration according to the present invention can be used for tissue regeneration, fillers, volumizers, restorative composites, etc. for the treatment of deep nasolabial folds, infraorbital acne/trauma scarring, nasolabial folds, oral commissure, mental creases, jawline contouring, glabellar lines, nasal defects, cheekbone, nasal tip, marionette lines, clefts, etc. in various fields such as dermatology, plastic surgery, dentistry, surgery, orthopedics, urology, otolaryngology, obstetrics & gynaecology, etc.

In the present invention, the term "tissue regeneration" refers to the action to restore tissue damaged by a physical cause or an aging or scar site to a normal state or a state close to normal and includes the meanings of "tissue healing", "tissue reconstruction" and "tissue recovery". In the present invention, the term "tissue damage" includes open wounds and closed wounds and may include wounds caused by invasive surgery applied to human or animal body, such as wounds caused by accidents, wounds caused by skin grafts, etc.

In the present invention, the term "scaffold for tissue regeneration" refers to a structure to be transplanted into a damaged tissue in the body or its periphery to regenerate the damaged tissue and then separated or completely degraded to disappear. The scaffold for tissue regeneration may be transplanted in vivo after inducing in vitro differentiation or may be transplanted in vivo, without including differentiation, to induce in vivo differentiation. After the scaffold for tissue regeneration is transplanted into the body, it is supplied with oxygen and nutrients from body fluids until new blood vessels are formed or supplied with blood through angiogenesis to proliferate and formed as a new tissue or organ.

The scaffold for tissue regeneration of the present invention may further comprise a biocompatible carrier and may be administered into tissue. The carrier is composed of substances, which have no toxicity and/or rejection to an object or have no side effects due to irritation, and acts as a collector or support such that cells cannot diffuse away from the transplant site. Moreover, it is apparent that the components and composition of the carrier can be easily determined by those skilled in the art that perform transplant operations. The biocompatible carrier may include, but not limited to, cell culture, hyaluronic acid, collagen, thrombin, elastin, chondroitin sulfate, albumin, etc.

The scaffold for tissue regeneration of the present invention may be transplanted by those skilled in the art and may be applied to body parts that are in need of tissue regeneration. The scaffold for tissue regeneration of the present invention may preferably be administered by parenteral administration such as intravenous administration, intraperitoneal administration, intramuscular administration, subcutaneous administration, intradermal administration, topical administration, etc., more preferably by subcutaneous administration or topical administration. Furthermore, it may be administered by skin application.

After the scaffold for tissue regeneration is administered into tissue, the tissue may be left untreated for a predetermined time such that the scaffold can be stably settled in the tissue, and then another scaffold for tissue regeneration may be administered, if necessary. The non-treatment period after the transplantation may be several days to several months, but the frequency and period may be adjusted by the knowledge of those skilled in the art depending on the condition of the subject being treated.

In order to maximize the efficiency of the scaffold for tissue regeneration of the present invention, it is preferable to apply a mechanical stress for the formation of the scaffold for tissue regeneration and apply the scaffold for tissue regeneration to an object after 72 hours. After 72 hours from the application of the mechanical stress, the production of extracellular matrices is maximized, and the production of fibroblasts is induced, thus exhibiting excellent tissue regeneration effect.

### [Mode for Invention]

In the following, the present invention will be described in detail with reference to the following Examples.

### Example 1: Isolation of adipose-derived mesenchymal stem cells

Mesenchymal stem cells were isolated from subcutaneous adipose tissue. Specifically, subcutaneous adipose tissue was separated, washed, and centrifuged. The subcutaneous adipose tissue was placed in a digestion solution (0.1% collagenase type I (GIBCO, USA), 0.001% Deoxyribonuclease I (DNase I ; Sigma-Aldrich, St. Louis, MO)) dissolved in Hank's balanced saline solution (HBSS, WelGENE, Korea) corresponding to 1/2 of the fat weight, and then the tissue was cut into pieces with scissors and applied with shear stress using a syringe. The tissue pieces were plated into tubes, stirred and incubated in a 37 °C incubator, and shaken every 5 minutes. The fat taken from the incubator was filtered using a 100 µm mesh in a pore-size dependent manner (100, 70, 50, 20, BD Falcon, Bedford MA, USA). The filtered fat was centrifuged at 400 g for 5 minutes, and the adipose tissue in the upper layer was removed. HBSS solution containing 10% fetal bovine serum (FBS, Hyclone, Canada) was added to deactivate the digestion solution, and then the resulting solution was centrifuged at 400 g for 5 minutes. The collected pellets were dissolved in DMEM (Dulbecco's modified Eagle's Medium, WelGENE, Korea) containing 1% antibiotic-antimycotic solution (GIBCO, Grand Island, NY) and 20% FBS, and then mesenchymal stem cells were seeded in a 35 mm dish (SPL, Korea).

### Example 2: Proliferation of mesenchymal stem cells

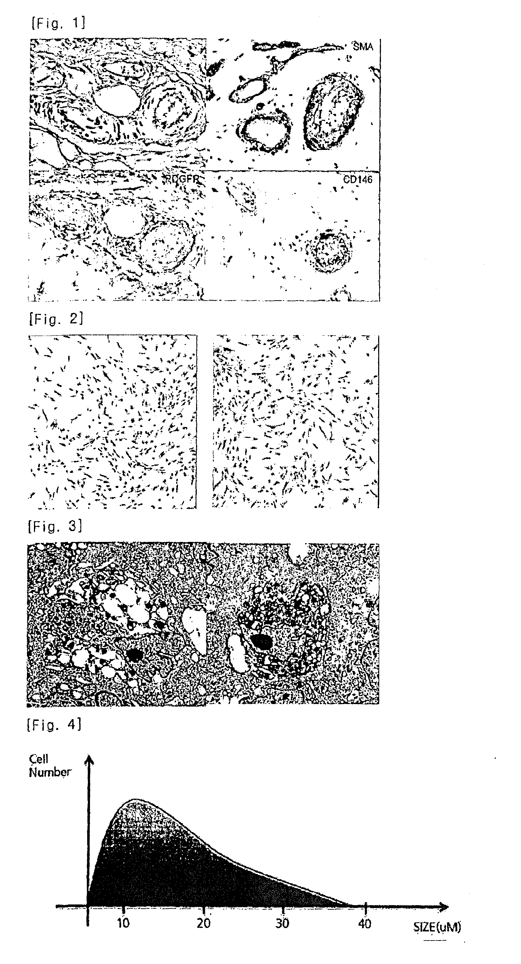

The mesenchymal stem cells obtained in Example 1 were incubated in DMEM containing 20% FBS (Hyclone, Canada) and 1% antibiotic-antimycotic solution in a 37 °C, 5% CO₂ incubator. At least one of FGF, EGF, PDGF or TGF-β was added for their proliferation efficiency. The medium was replaced every two days, and the cells were passaged at a confluency of 70% using 0.25% trypsin-EDTA (GIBCO, Grand Island, NY). The adipose-derived mesenchymal stem cells obtained through the above process were stained with different markers, and the adipose-derived mesenchymal stem cells at passages 1 and 2 were observed. The results are shown in FIGS. 1 to 4.

As shown in FIG. 1, the adipose-derived mesenchymal stem cells were positive to SMA, PDGFR, and CD146. Moreover, as shown in FIGS. 2 and 3, it was found that the cells proliferated normally as the passage continued. Furthermore, as shown in FIG. 4, the distribution of cell number according to cells size was determined.

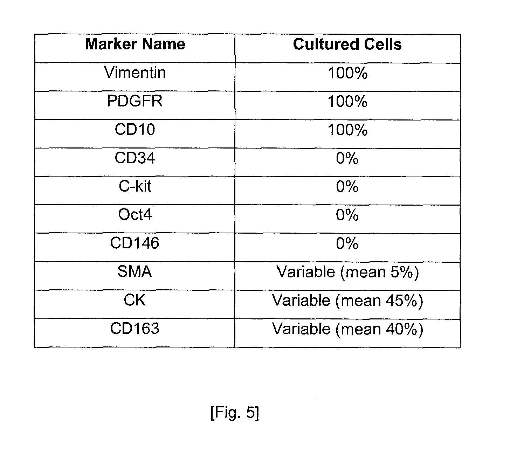

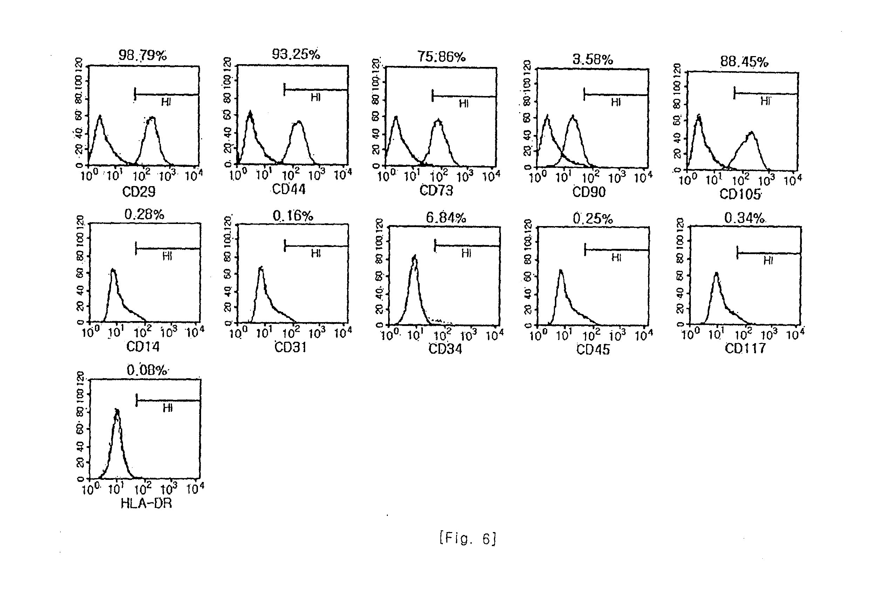

Moreover, the phenotype of adipose-derived mesenchymal stem cells was identified by immunohistochemical analysis, and the cell surface antigens on adipose-derived mesenchymal stem cells were determined by FACS analysis. The results are shown in FIGS. 5 and 6, respectively.

As shown in FIG. 5, it was found that the adipose-derived mesenchymal stem cells were positive to vimentin, PDGFR, and CD10 and negative to CD34, c-kit, Oct4, and CD146. Moreover, as shown in FIG. 6, it was found that the minimum required numbers of markers for adipose-derived mesenchymal stem cells, such as CD73, CD90, and CD105, were highly expressed, and CD28 and CD44 were also highly expressed. Contrary to this, it was found that the hematopoietic stem cell markers such as CD14, CD34, and CD117, the vascular endothelial cell marker such as CD31, and the blood cell markers such as CD45 and HLA-DR were almost not expressed. The above results confirmed that the adipose-derived mesenchymal stem cells isolated in Example 1 have the characteristics of mesenchymal stem cells.

### Example 3: Preparation of scaffolds for tissue regeneration through induction of differentiation of mesenchymal stem cells

The mesenchymal stem cells, obtained in Example 2, at passage 7 cultured on a single layer were treated with 0.25% trypsin-EDTA. The following three methods were used to prepare three-dimensional clusters (scaffolds) by inducing differentiation of mesenchymal stem cells.

The first method was to use hanging drop to apply a force of less than 1G. More specifically, cells were suspended in a 25 µl medium and plated on a coverslip, and the coverslip was carefully turned upside down. Then, the bottom of the dish was covered with deionized water such that the medium of cells cultured in hanging drops were not dried. After 2 to 3 days, the three-dimensional cluster (scaffold) formed was transferred to a microplate. The second method was performed in such a manner that cells were placed into 15 ml tubes and centrifuged at 50 g for 5 minutes without deceleration to induce mesenchymal condensation, followed by culture. The third method was performed in such a manner that cells were placed into 15 ml tubes and centrifuged at 500 g for 5 minutes, followed by culture.

The scaffolds (pellets) obtained according to the respective methods were incubated in a 37 °C, 5% CO₂ incubator for several days to several weeks depending on desired size of the scaffold, and the medium was replaced every three days.

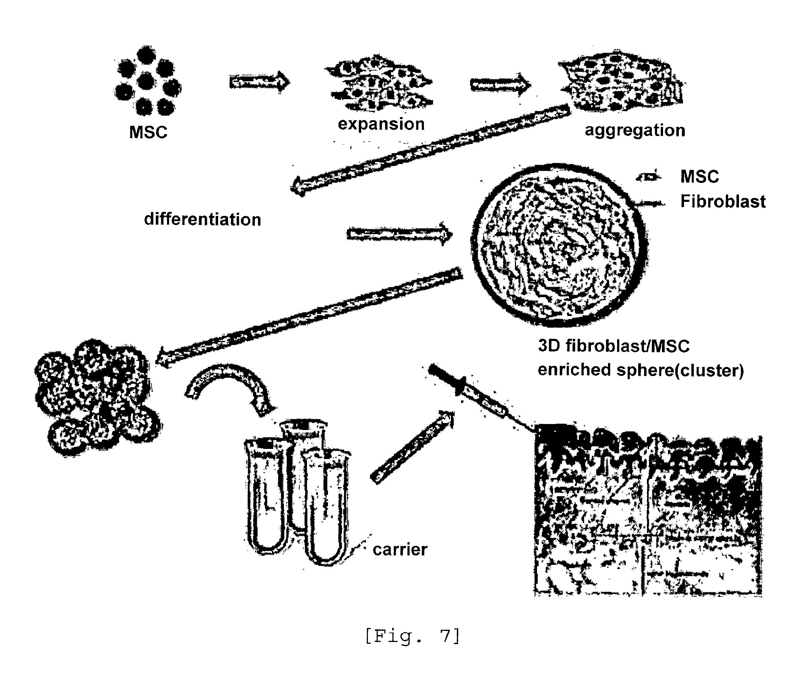

The above-described method for preparing the scaffold for tissue regeneration is shown in the schematic diagram of FIG. 7.

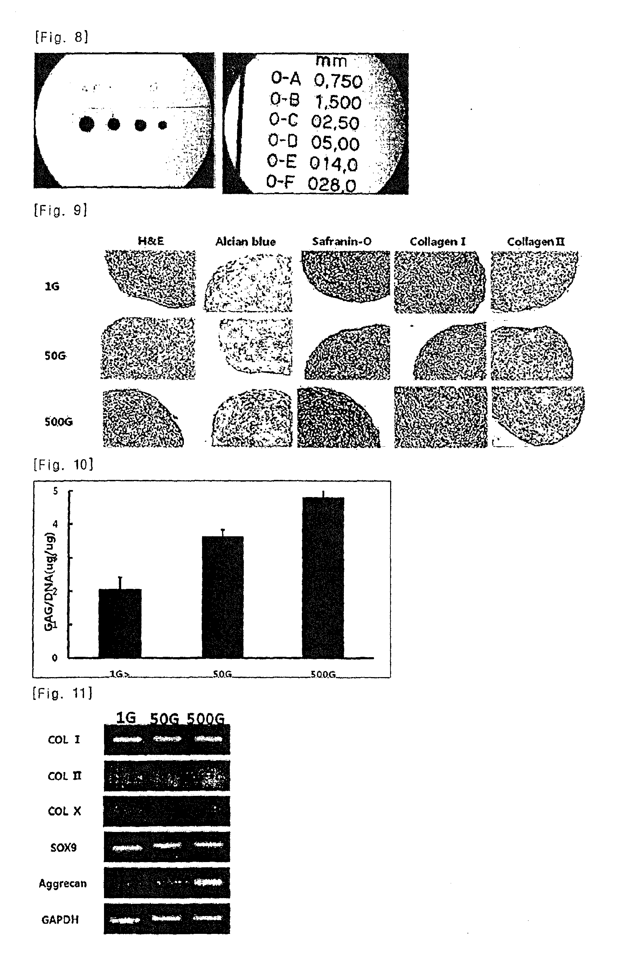

The morphologies of the scaffolds for tissue regeneration as the differentiated derivatives of mesenchymal stem cells obtained through the above process are shown in FIG. 8.

As shown in FIG. 8, it was found that the differentiated derivatives of mesenchymal stem cells according to the present invention could be prepared with a diameter between several micrometers to several centimeters by controlling physical force and adjusting culture period, without the use of any additional substances such as biopolymers.

### Example 4: Characterization of scaffolds for tissue regeneration through induction of differentiation of mesenchymal stem cells

In order to characterize the differentiated derivatives of mesenchymal stem cells obtained in Example 3, the obtained scaffolds were fixed in 4% formalin (Junsei, japan) for 24hr and embedded in paraffin. Then, in order to identify the components of the scaffolds according to the present invention, the scaffolds were stained with hematoxylin and eosin (H&E, Sigma-Aldrich, St. Louis, MO), Alcian blue (Wako), Safranin-O/fast green (Fisher, New Jersey), Collagen I, and Collagen II. The results are shown in FIG. 9.

As shown in FIG. 9, it was found that the scaffolds for tissue regeneration as the differentiated derivatives of mesenchymal stem cells according to the present invention contained extracellular matrices such as mucin, GAG, etc. (when stained with Alcian blue and Safranin-O) and produced Collagen I rather than Collagen II. Moreover, pathological diagnosis of the differentiated derivatives of mesenchymal stem cells observed with the staining revealed that when the differentiated derivatives of mesenchymal stem cells according to the present invention were cultured in vitro for several weeks, approximately 10% cells differentiated into fibroblasts. The above results confirmed that when the scaffold of the present invention is transplanted in vivo, the mesenchymal stem cells in the scaffold differentiate into fibroblasts, and thus the scaffold of the present invention can exhibit an excellent effect when it is used as a filler, volumizer, etc.

### Example 5: Glycosaminoglycan (GAG) assay

Blyscan Sulfated Glycosaminoglycan Assay (biocolor, Belfast, Ireland) was performed to measure the amount of extracellular matrix, glycosaminoglycan (GAG), in the differentiated derivatives of mesenchymal stem cells obtained in Example 3. More specifically, the scaffold was placed in EP tube, and 500 µl solution containing 10 µl/ml papain (Sigma-Aldrich, St. Louis, MO, USA) in papain buffer solution (papain buffer solution, 100ml of 0.2M sodium phosphate buffer, add 0.1M sodium acetate, 10nM EDTA, 5mM L-cysteine HCl, pH 6.4) was added thereto. The mixture was incubated in a 60 °C incubator for 24 hours and centrifuged at 3,300 g for 5 minutes.

The supernatant and 1 mL of dry reagent were added to the mixture and left at room temperature for 30 minutes. Then, the supernatant was removed by centrifugation at 10,000 g for 10 minutes, and the pellets were dried at room temperature. 1 mL of dissociation reagent was added to dissolve the pellets, and the cells were plated in a 96-well microplate (NUNC, Denmark). Then, the absorbance was measured at 656 nm using ELISA reader. Chondroitin-4-sulfate was used as a standard, and the GAG was normalized to total DNA content. The total DNA content was assessed with the Quant-iT PicoGreen dsDNA Assay Kit (Invitrogen, USA). The results are shown in FIG. 10.

As shown in FIG. 10, it was found that the scaffold for tissue regeneration as the differentiated derivatives of mesenchymal stem cells according to the present invention contained a large amount of GAG, and in particular, as the G value increased, the production of GAG increased.

### Example 6: Gene analysis

Analysis of genes in the differentiated derivatives of mesenchymal stem cells obtained in Example 3 was performed. The results are shown in FIG. 11.

As shown in FIG. 11, the expression of Collagen I, SOX9, aggrecan, etc. was observed high in the scaffold for tissue regeneration as the differentiated derivatives of mesenchymal stem cells according to the present invention, but the expression of Collagen II and Collagen X was observed low. This confirmed that the differentiated derivatives of mesenchymal stem cells according to the present invention serve to promote the production of extracellular matrices during the differentiation.

The invention has been described in detail with reference to preferred embodiments thereof. However, it will be appreciated by those skilled in the art that changes may be made in these embodiments without departing from the principles and spirit of the invention, the scope of which is defined in the appended claims and their equivalents.

## Claims

1. A method for preparing a scaffold for tissue regeneration, the method comprising the steps of:
(a) inducing aggregation of mesenchymal stem cells by applying a mechanical stress to the mesenchymal stem cells; and
(b) obtaining a scaffold containing both differentiated derivatives in a spherical form and mesenchymal stem cells by culturing the aggregated mesenchymal stem cells.

2. The method of claim 1, wherein the mesenchymal stem cells are derived from adipose tissue, umbilical cord, or bone marrow.

3. The method of claim 1, wherein the mesenchymal stem cells are autologous or allogeneic.

4. The method of claim 1, wherein the scaffold for tissue regeneration has a diameter of 1 µm to 10 cm.

5. The method of claim 1, wherein in step (b), the differentiated derivatives comprise fibroblasts.

6. The method of claim 1, wherein in step (b), a bioactive substance is further added during the culture.

7. The method of claim 6, the bioactive substance comprises at least one additive selected from the group consisting of fibroblast growth factor (FGF), epidermal growth factor (EGF), platelet-derived growth factor (PDGF), connective tissue growth factor (CTGF), bone morphogenic protein (BMP), lipopolysaccharide (LPS), tumor necrosis factor-α (TNF-α), transforming growth factor-α (TGF-α), transforming growth factor-β (TGF-β), ascorbic acid, and dexamethasone.

8. A scaffold for tissue regeneration prepared by the method of any one of claims 1 to 7, the scaffold having a diameter of 1 µm to 10 cm and containing both mesenchymal stem cells and differentiated derivatives thereof.
